(19) Europäisches Patentamt
European Patent Office
Office européen des brevets

(11) Publication number: 0 337 803 A1

(12) **EUROPEAN PATENT APPLICATION**

(21) Application number: 89303718.4

(22) Date of filing: 14.04.89

(51) Int. Cl.[4]: B 01 J 31/40
C 07 C 57/30, C 07 C 51/12

(30) Priority: 15.04.88 US 182263

(43) Date of publication of application:
18.10.89 Bulletin 89/42

(84) Designated Contracting States:
AT BE CH DE ES FR GB GR IT LI LU NL SE

(71) Applicant: HOECHST CELANESE CORPORATION
Route 202-206 North
Somerville, N.J. 08876 (US)

(72) Inventor: Mott, Graham N.
5309 Wentworth Drive
Corpus Christi Texas (US)

Zey, Edward G.
522 Evergreen
Corpus Christi Texas (US)

(74) Representative: De Minvielle-Devaux, Ian Benedict Peter et al
CARPMAELS & RANSFORD 43, Bloomsbury Square
London WC1A 2RA (GB)

(54) **Catalyst recycle in the carbonylation of isobutylphenylethanol to ibuprofen.**

(57) In the carbonylation of isobutylphenyl ethanol to ibuprofen in the presence of a phosphine ligand-stabilized palladium catalyst, it has been found that the catalyst complex will precipitate from the organic phase of the carbonylation reaction which includes the ibuprofen product. Precipitation of the catalyst is facilitated by removing the aqueous phase from the reaction and removing any solvents from the organic phase. The precipitated complex catalyst can be recycled without further treatment.

EP 0 337 803 A1

Description

# CATALYST RECYCLE IN THE CARBONYLATION OF ISOBUTYLPHENYLETHANOL TO IBUPROFEN

## FIELD OF THE INVENTION

This invention is directed to a method of recovering and recycling noble metal catalyst complexes useful in the carbonylation of isobutylphenylethanol to 2-(4′-isobutylphenyl)propionic acid, i.e., ibuprofen. More particularly, the invention concerns the recovery and recycle of phosphine ligand-stabilized palladium halide complexes used in the carbonylation of isobutylphenylethanol to ibuprofen.

## BACKGROUND OF THE INVENTION

Ibuprofen is a well-known non-steroidal anti-inflammatory drug which has been converted from ethical, i.e., prescription, to over-the-counter status. Various processes are known for the production of iboprofen using 4-isobutylacetophenone as a starting material. Thus, for example, British Patent No. 971,700 and corresponding U.S. Patent No. 3,385,886, both assigned to Boots Pure Drug Company, PLC, show the production of phenylalkane derivatives such as ibuprofen in which the first step of the process is the reaction of an alkylbenzene with acetyl chloride in the presence of aluminum chloride to produce an alkylacetophenone which is then subjected to any of various series of subsequent reactions to produce the desired derivative.

Arylpropionic acids, in general, have been formed by the carbonylation of the respective arylethyl alcohol. For example, Japanese Kokai Patent No. SHO 55 [1980]-27147, published February 27, 1980 and assigned to Mitsubishi Petrochemical Co., discloses the formation of ibuprofen by reacting 1-(4′-isobutylphenyl)ethanol with carbon monoxide and water in the presence of a hydrogen fluoride catalyst. Japanese Kokai Patent No. SHO 59 [1984]-95238, published June 1, 1984 and assigned to Mitsubishi Petrochemical Co., teaches the formation of alpha-aryl-substituted propionic acids, by reacting an alpha-aryl substituted ethanol with carbon monoxide and water, alcohol, or phenol, in the presence of a palladium catalyst. An acidic compound such as hydrogen chloride may be added as an auxiliary catalyst and a solvent such as benzene may also be used. The disclosure includes a comparative example in which ibuprofen (not included within the invention) is obtained in very low yield, i.e., 17.1%, when made utilizing the described process. Japanese Kokai Patent No. SHO 59 [1984]-95239, published June 1, 1984 and assigned to Mitsubishi Petrochemical Co., discloses the formation of alpha-(6-methoxy-2-naphthyl) propionic acid by reacting alpha-(6-methoxy-2-naphthyl)ethyl alcohol with carbon monoxide and water in the presence of a palladium catalyst and an acidic compound, e.g., hydrogen chloride. The patent publication also states that if a non-halogen-containing acidic compound is used, it is desirable to add an ionizable metal halide to the reaction.

Japanese Kokuku Patent No. SHO 56[1981]-35659, published September 4, 1978 and assigned to Ferrel International Societe Annonim, discloses an anhydrous method of producing a 2-(4′-isobutylphenyl)propionic acid ester by treating 1-(4′-isobutylphenyl)ethanol (IBPE) with carbon monoxide in a solution containing an alkanol and a catalyst such as palladium bis(triphenylphosphine) dichloro complex. The solution may also contain up to 10% of a mineral acid such as hydrogen chloride.

British Patent 1,565,235, assigned to Mitsubishi Petrochemical Co. discloses a process for producing alpha-aryl-propionic acids which have an anti-inflammatory, analgesic or antipyretic effect. The process comprises reacting an arylethylene with carbon monoxide under pressure in the presence of a carbonylation catalyst and in the presence of water and/or a lower alcohol to carbonylate the arylethylene into the alpha-aryl propionic acid. The starting materials for the arylethylenes can be prepared such as by the dehydration of arylethyl alcohols or by the dehydrohalogenation of arylethyl halides. The arylethylenes can be purified by means of a single distillation or re-crystallization to obtain the products with a sufficiently high purity to be used as the starting material for the subsequent carbonylation step.

In copending, commonly assigned U.S. Serial No. 158,141, filed March 4, 1988, 2-(4′-isobutylphenyl)propionic acid, i.e., ibuprofen, is prepared by carbonylating 1-(4′-isobutylphenyl)ethanol (IBPE) with carbon monoxide in an acidic aqueous medium at a temperature of at least about 10°C and a carbon monoxide pressure of at least about 500 psig, and in the presence of 1) a catalyst consisting essentially of a palladium compound in which the palladium is complexed with at least one acid stable, monodentate phosphine ligand freely miscible with the organic phase of the reaction medium; 2) dissociated hydrogen ions from an acid which is substantially completely ionizable in a dilute aqueous solution such that the molar ratio of hydrogen ions to IBPE added to the reaction zone is at least about 0.15 and the mole ratio of hydrogen ions to water is at least 0.026; and 3) dissociated halide ions such that the molar ratio of halide ions to IBPE added to the reaction zone is at least about 0.15.

The process as immediately described above for preparing ibuprofen by carbonylating 1-(4′-isobutylphenyl)-ethanol (IBPE) is generally a multi-stage process in which the carbonylation reaction to produce ibuprofen is integrated with a method of producing IBPE from isobutylbenzene. Thus, isobutylbenzene is subjected to Friedel-Crafts reaction with an acetylating agent such as acetic acid, acetic anhydride, acetyl fluoride, acetyl

chloride, acetyl bromide, methyl acetate in the presence of the Friedel-Crafts catalyst such as hydrogen fluoride to produce 4-isobutylacetophenone (IBAP) which is subsequently reduced with hydrogen in the presence of a hydrogenation catalyst or with a reducing agent containing available hydrogen, to obtain IBPE.

In view of the expense of the carbonylation catalyst used, e.g., palladium, to produce ibuprofen from IBPE, it would obviously be worthwhile to collect and recycle the catalyst for use in the carbonylation reaction. Recovery and recycle of a sufficiently pure palladium catalyst has not yet been achieved. Typically, a sufficient amount of a catalyst sludge is eventually formed and is returned to the reactor, see U.S. 3,455,989. However, as disclosed in U.S. 4,013,583, a palladium complex catalyst used in the carbonylation, hydroformylation and hydrogenation of olefins has been recovered and regenerated. Recovery of the palladium carbonylation catalyst is achieved by dispersing the ligand-stabilized palladium halide catalyst in quaternary ammonium, phosphonium or arsonium salts of trihalostannate or trihalogermanate. Thus, the palladium catalyst recovered contains the stannate or germanate component.

In the carbonylation of IBPE to ibuprofen, it would be worthwhile to recover the palladium catalyst complex in relatively pure form from the iboprofen reaction products. Similarly, it would be highly advantageous to maintain high conversion, high selectivities and high yields even after recycling the catalyst several times. It would also be useful to recover the catalyst by a means which does not degrade or decompose the catalyst. This is particularly important in recovering ligand-stabilized palladium catalysts which are thermally unstable.

## SUMMARY OF THE INVENTION

It has now been discovered that in the carbonylation of 1-(4′-isobutylphenyl)ethanol (IBPE) to 2-(4′-isobutylphenyl)propionic acid, i.e., ibuprofen, in an acidic aqueous medium, the carbonylation catalyst consisting essentially of palladium halide, complexed with a triorganophosphine ligand, can be precipitated from the reaction products in substantially pure form and recycled directly to the carbonylation reactor. Precipitation of the catalyst is not induced by the addition of extraneous agents as in U.S. 4,013,583, which agents may adversely effect catalysis and the nature of the product formed.

Thus, in accordance with the present invention, ibuprofen is prepared by carbonylating 1-(4′-isobutylphenyl)ethanol with carbon monoxide under the process conditions set forth in aforementioned commonly assigned U.S. Serial No. 158,141 in which the carbonylation catalyst is palladium halide complexed with triorganophosphine. The carbonylation reaction takes place at a temperature of at least about 10°C and a carbon monoxide pressure of at least about 500 psig and in the presence of 1) the palladium complex catalyst; 2) dissociated hydrogen ions such that the molar ratio of hydrogen ions to IBPE added to the reaction zone is at least about 0.15; 3) dissociated halide ions such that the molar ratio of halide ions to IBPE present in the reaction zone is at least about 0.15 and 4) optionally an organic solvent. Upon completion of the reaction, two phases are formed, an aqueous phase containing the dissociated hydrogen and halide ions and an organic phase containing the ibuprofen, byproducts and solvent, if used. The aqueous phase is removed by known separatory techniques and returned to the reactor. Upon removal of any solvent from the organic phase, the palladium halide complex catalyst precipitates from the organic phase in substantially pure form and can be collected and recycled directly to the carbonylation reactor.

## DETAILED DESCRIPTION OF THE INVENTION

The carbonylation reaction proceeds in accordance with equation (I):

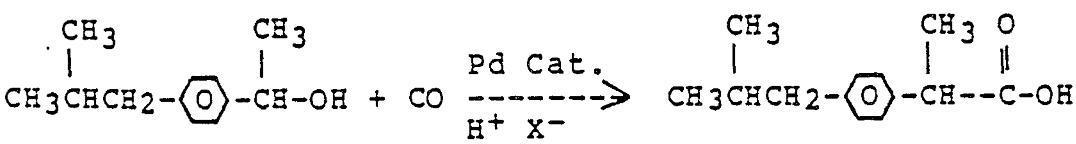


(I)

wherein X is chlorine, bromine, or iodine.

In carrying out the carbonylation reaction, water may be present in an amount, for example, of about 10 to 600%, preferably about 15 to 100%, based on the weight of IBPE initially present; the temperature of reaction may be, for example, in the range of about 10 to 225°C, preferably about 70 to 175°C; the carbon monoxide pressure may be, for example in the range of about 500 to 5000 psig, preferably about 700 to 3000 psig; and the total reaction time may be, for example, in the range of about 0.1 to 24 hours, preferably about 1 to 6 hours.

The palladium catalyst which is used is complexed with at least one acid stable monodentate phosphine ligand freely miscible with the organic phase of the reaction medium. Suitable examples wherein palladium is complexed with an appropriate phosphine ligand are as follows:
bis(triphenylphosphine) dichloro complex, bis(tributylphosphine) dichloro complex, bis(tricyclophenylphosphine) dichloro complex, pi-allyltriphenylphosphine dichloro complex, triphenylphosphine piperidine dichloro complex, bis(triphenylphosphine) dicarbonyl complex, bis(triphenylphosphine) diacetate complex, bis(triphenylphosphine) dinitrate complex, bis(triphenylphosphine) sulfate complex, tetrakis(triphenylphosphine) complex, and complexes in which some of the ligands are carbon monoxide such as chlorocarbonyl

bis(triphenylphosphine) complex, all complexes of palladium.

The palladium salts and phosphine ligands making up the foregoing catalyst complexes may also be added separately to the reaction zone. In this case, the amount of ligand added is preferably sufficient to complex with the palladium present such that the P:Pd mole ratio is equal to at least about 1:1 when the Pd:IBPE mole ratio is at least about 1:5,000. However, when the latter ratio is below about 1:10,000, it is necessary to use an excess of phosphine ligand such that the P:Pd ratio is at least about 2:1.

The catalyst complex may be present in an amount such that the mole ratio of palladium to IBPE is in the range, for example, of about 1:25 to 1:20,000, preferably about 1:150 to 1:10,000, and most preferably about 1:1,000 to 1:6,000.

The dissociated hydrogen ions and halide ions may be conveniently added to the reaction as hydrogen halide, e.g., hydrogen chloride, hydrogen bromide, or hydrogen iodide. However, it is also possible to add the hydrogen ions and halide ions from separate sources. For example, other acids completely ionizable in dilute aqueous solution, e.g., inorganic acids, such as sulfuric acid, phosphoric acid or polyphosphoric acid, or organic acids, e.g., sulfonic acids such as p-toluenesulfonic acid, methanesulfonic acid or trifluoroacetic acid, may be used as the source of hydrogen ions. Similarly, other water-soluble and ionizable halide compounds, as for example, halide salts wherein the cation does not interfere with the reaction, e.g., alkali metal halides such as potassium, sodium, and lithium chlorides, bromides, and iodides may be used as the source of halide ions. The molar ratio of hydrogen ions and halide ions to IBPE ($H^+$/IBPE and $X^-$/IBPE) each may be in the range, for example, of about 0.15 to 5, preferably about 0.3 to 2.0.

Although not necessary for the operability of the process, in some instances, it may be advantageous to utilize an organic solvent for the reaction. Organic solvents which can be used in the process of this invention are, for example, ketones such as methyl ethyl ketone, acetone, 2-pentanone, 3-pentanone, and acetophenone, aromatic hydrocarbons such as benzene and toluene, and cyclic ethers such as tetrahydrofuran and dioxane. Ketones are ethers are the preferred solvents. Certain solvents, e.g., ketones, may also serve as the ligand forming a complex with the palladium. If the catalytic palladium as added to the system is in the metallic or zero valence state ($Pd^\circ$), then any solvent used should be non-hydrocarbon. The solvent, if used, may be present in a weight ratio of solvent to IBPE in the range, for example, of about 0 to 1000:1, preferably about 0 to 10:1.

An inorganic salt may also be present during the reaction. Inorganic salts which may be used are, for example, those yielding anions comprising oxygen, and sulfur, phosphorus, aluminum or silicone, including such anions as hydrogensulfate, pyrosulfate, ortho-phosphate, pyrophosphate, aluminate, or silicate and cations such as sodium, potassium, calcium, or magnesium, or another cation which does not interfere with the reaction, e.g., ammonium or alkylammonium such as tetrabutylammonium. Other inorganic salts such as calcium chloride may also be added. The inorganic salt, if used, will generally be present at a concentration of, for example, about 0.1 to 50%, preferably about 1 to 20% by weight of total charge.

In some instances, an undesirable heavy ends fraction may form during the reaction, possibly due to a polymerization mechanism of unknown nature. In view of this, it may be beneficial to incorporate a polymerization inhibitor in the reaction mass. Inhibitors which may be used for this purpose include, for example, t-butylcatechol, hydroquinone, m-dinitrobenzene, N-nitrosodiphenylamine, picric acid, sodium sulfite, quinhydrone, and the like. If an inhibitor is utilized, it may be incorporated in an amount, for example, of about 0.01 to 15%, preferably about 0.1 to 5% by weight based on the weight of IBPE.

In addition to those mentioned previously, other additives and ligands may be added to the reaction, e.g., acetophenone and p-mercaptoacetophenone. The latter additives appear to be useful in raising the ratio of ibuprofen to corresponding linear isomer, viz., 3-(4′-isobutylphenyl)propionic acid (3-IPPA), obtained by the method of this invention.

The IBPE used to produce ibuprofen in accordance with the method of this invention may be made by any of various means. Preferably, however, the carbonylation reaction to produce ibuprofen is integrated with a method of producing IBPE from isobutylbenzene wherein the later compound is subjected to Friedel-Crafts reaction with an acetylating agent to produce 4′-isobutylacetophenone (IBAP) which is then reduced with hydrogen in the presence of a hydrogenation catalyst, or with a reducing agent containing available hydrogen, to obtain IBPE.

The Friedel-Crafts acetylation of isobutylbenzene to produce 4′-isobutylacetophenone proceeds in accordance with equation (II):

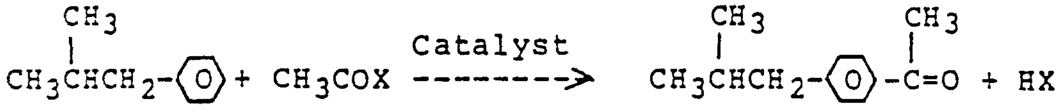

(II)

where X is the residue minus an acetyl group of compounds which are known acetylating agents. X may be, for example, hydroxy, acetoxy, or halide including chloride, fluoride, or bromide. Acetylating agents which may be used are for example acetic acid, acetic anhydride, acetyl fluoride, acetyl chloride, acetyl bromide, methyl acetate, and ketone, which results from the abstraction of HX from the foregoing acetylating agents prior to the acetylation reaction. The acetylating agent may be used in an amount, for example, of about 1 to 4 moles, preferably about 1.1 to 2.0 moles per mole of isobutylbenzene employed.

The Friedel-Crafts catalyst may be hydrogen fluoride or any other catalyst known in the art to be effective for the Friedel-Crafts reaction, e.g., aluminum chloride, zinc chloride, or boron trifluoride. In carrying out the reaction, the isobutylbenzene, acetylating agent, and catalyst, may be charged to a corrosion-resistant reactor and the mixture maintained at a temperature, for example, of about 0 to about 120° C, for a period, for example, of about 0.5 to about 5 hours. The pressure is uncritical and atmospheric or autogenous pressure may be utilized. If HF is used as the catalyst, it may be charged as a liquid or a gas using technologies of handling well-known to those skilled in the art. In carrying out the reaction, an inert gas such as nitrogen may be used to keep sufficient HF in contact with the reacting liquid. An excess of HF is generally used, for example, about 10 to 100 moles, preferably about 25 to about 75 moles per mole of isobutylbenzene initially present in the reaction zone.

The hydrogenation or reduction of IBAP to form IBPE proceeds in accordance with equation (III) where "[H]" represents the available hydrogen in hydrogen gas in the presence of a hydrogenation catalyst or in a hydrogen-containing reduction agent such as sodium borohydride or lithium aluminum hydride:

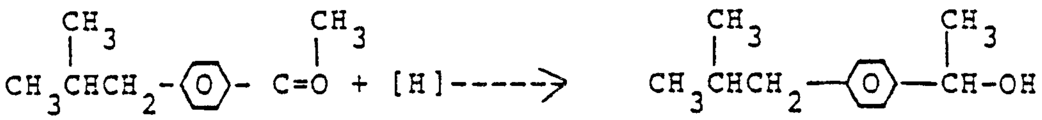

(III)

The hydrogenation or reduction as shown in equation (III) may be accomplished, for example, by contacting IBAP as is or dissolved in an appropriate solvent with a hydrogenation catalyst in the presence of hydrogen. The solvent may be, for example, methanol, ethanol, t-butanol, aqueous alcohol, toluene, diethyl ether, tetrahydrofuran, or 1,4 dioxane, and the IBPE: solvent weight ratio may be in the range, for example of about 1:1 to 1:100, preferably about 1:2 to 1:20. The hydrogenation catalyst may be, for example, a transition metal on a suitable support. Preferred transition metals are nickel, e.g., Raney nickel, and the noble metals, e.g., palladium, platinum, rhodium, iridium, ruthenium, and osmium, and some suitable supports are, for example, carbon, alumina, silica, and polymeric resins. The metal concentration on the support in weight ratio of metal:support may be in the range, for example, of about 1:100 to 1:2, preferably about 1:50 to 1:10, and the weight ratio of catalyst system:IBAP is, for example, in the range of about 1:500 to 1:2, preferably about 1:30 to 1:5. In carrying out the reaction, the hydrogen pressure may be in the range, for example, of about 10 to 1200 psig, preferably about 75 to 300 psig; the reaction temperature may in the range, for example, of about 10 to 150° C, preferably about 20 to 80° C; and the reaction time may be in the range, for example, of about 0.25 to 10.0 hours, preferably about 1.0 to 4.0 hours. Under some conditions, the addition of a base may be desirable to prevent hydrogenolysis.

Alternative to the hydrogenation reaction as described, the reduction reaction shown in equation (III) may be accomplished, for example, by slowly adding to a cooled solution of IBAP in an alcohol, e.g., methanol, ethanol, or t-butanol, or an ether such as tetrahydrofuran or diethyl ether, a reducing agent containing available hydrogen, e.g., sodium or potassium borohydride or lithium aluminum hydride. The solution may then be warmed to room temperature and heated at reflux, e.g., for a period of about 0.5 to 3.0 hours.

In accordance with the present invention, the palladium complex catalyst used in the carbonylation of IBPE to ibuprofen according to equation (I) is precipitated in substantially pure form and can be collected for recycle to the carbonylation reaction. It is believed that the unique mixture of carboxylic acids including ibuprofen in the product, and the remaining organic medium such as solvent allows for the formation and ultimate precipitation of the phosphine ligand-stabilized palladium complex catalyst without the addition of a separate complexing agent which may alter in a significant way the chemical composition of the catalyst complex.

Upon completion of the carbonylation reaction such as by equation (I), a reaction product is formed which contains two separate liquid phases. The heavier aqueous phase contains the dissociated hydrogen and halide ions while the lighter organic phase contains the ibuprofen product, organic by-products, any solvent and the soluble palladium complex catalyst. To recover the catalyst, the aqueous and organic phases must be separated. Separation of the phases can be done by well known techniques such as by decantation from a separatory funnel and the like. If the phases are not well defined on completion of the carbonylation reaction, an organic solvent can be added to improve phasing. For example, in a reaction medium which contains a ketone solvent such as methylethyl ketone, the addition of ethyl acetate has been found to greatly improve the phase separation between the aqueous and organic phases. The amount of phasing organic solvent added to enhance phase separation can vary widely but, will typically range from about 0.1 to 10 parts volume per part volume of liquid reaction medium. The aqueous phase once separated can be returned directly to the carbonylation reactor.

If a solvent has not been used in the carbonylation reaction, the phosphine ligand-stabilized palladium catalyst will precipitate out of the organic phase in essentially pure form. This precipitate can be collected and recycled directly to the carbonylation reactor. No treatment of the catalyst precipitate is needed prior to recycle.

If a solvent has been used in the carbonylation reaction, the solvent must be removed before precipitation takes place. Removal of solvent from the organic phase can be done in various ways such as by extraction or by distillation. Further, certain solvents useful in the carbonylation are good coordinating solvents and serve as

ligands which form a tightly bound complex with the palladium catalyst. Ketones are examples of good coordinating solvents. Thus, if a coordinating solvent has been used, a nonsolvent or noncoordinating solvent can be added to disengage the coordinating solvent from the palladium phosphine complex catalyst. Upon addition of the nonsolvent, the palladium phosphine complex catalyst precipitates from the organic phase. The amount of noncoordinating solvent added will also vary widely depending on the coordinating and noncoordinating solvents used. In general, about 0.1 to 10 parts volume of noncoordinating solvent per part volume of organic phase will disengage the solvent ligand and provide for precipitation of the palladium-phosphine complex catalyst. An example of a useful solvent which coordinates with the palladium complex catalyst is methylethyl ketone. The addition of ethyl acetate to the organic phase containing the methylethyl ketone initiates precipitation of the phosphine ligand stabilized palladium halide complex catalyst. In each case, whether or not a solvent is present or whether the solvent is removed or a nonsolvent is added, nominal cooling of the organic phase may be required. Precipitation of the catalyst takes place at about 100° C and, thus some cooling may be required.

Carbonylation of IBPE to ibuprofen with the recycled aqueous phase and palladium catalyst does not require any change in process conditions. It is only necessary to add make-up hydrogen and halide ions to the aqueous phase and provide for make-up catalyst. It has been found that about 90% of the palladium catalyst will precipitate and can be recovered for recycle.

## EXAMPLE

The following ingredients were added to a 4 liter autoclave: 300 g (1.69 moles) 1-(4′-isobutylphenyl)ethanol, 840 mL methylethylketone, 2 g (0.0113 moles) palladium (II) chloride, 9 g (0.0344 moles) triphenyl phosphine, 38 g (0.34 moles) calcium chloride, 203 ml 36% HCl, 547 mL water. After flushing with nitrogen and carbon monoxide, the autoclave was pressured to approximately 700 psig of carbon monoxide. The reactor was stirred and heated to 130° C during which time the pressure inside the reactor increased to approximately 1000 psig and was subsequently maintained at this pressure during the 2 hour reaction time. After the carbon monoxide uptake was complete the reactor was cooled to room temperature and vented. The contents of the reactor were removed and the two phases were separated in a separatory funnel. The aqueous phase was extracted with 500 mL ethyl acetate and the organic fractions were combined and the solvents removed on a rotary evaporator under reduced pressure. During evaporation 6.4 g (81% of theoretical) yellow crystalline $PdCl_2(PPh_3)$ suitable for reuse, precipitated and was recovered. 0.2 g (16% of theoretical) of palladium metal was recovered by filtration of the aqueous phase. The organic product obtained was 348 g and contained 57% ibuprofen.

**Claims**

1. In a method of preparing ibuprofen comprising carbonylating 1-(4′-isobutylphenyl)ethanol (IBPE) with carbon monoxide in an acidic aqueous medium and in the presence of (1) a catalyst consisting essentially of a palladium compound which is complexed with a phosphine ligand; (2) dissociated hydrogen ions; (3) dissociated halide ions; and (4) optionally a solvent, the improvement which comprises: recovering the palladium-phosphine catalyst complex by precipitating said complex from the organic phase formed from said carbonylation in which said organic phase contains the ibuprofen product.

2. The improvement of claim 1 wherein the carbonylation product includes an aqueous phase containing said dissociated hydrogen and halide ions and wherein said aqueous phase is separated from said organic phase prior to precipitation of said catalyst.

3. The improvement of claim 2 wherein an organic agent is added to improve the phase separation between said organic and aqueous phases.

4. The improvement of claim 2 wherein no solvent is present in the organic phase.

5. The improvement of claim 2 wherein a solvent is present in the organic phase and removing said solvent prior to precipitation of said catalyst.

6. The improvement of claim 2 wherein said organic phase contains a coordinating solvent which forms a ligand with said palladium catalyst, precipitating said palladium complex catalyst by adding a noncoordinating solvent which disengages the coordinating solvent ligand from said palladium.

7. The improvement of claim 6 wherein the coordinating solvent is a ketone.

8. The improvement of claim 7 wherein said coordinating solvent is methylethyl ketone.

9. The improvement of claim 8 wherein said noncoordinating solvent is ethyl acetate.

10. The improvement of claim 2 wherein said organic phase has a temperature of no greater than about 100° C.

11. The improvement of claim 1 wherein said phosphine ligand is a triorganophosphine.

12. The improvement of claim 11 wherein said ligand is triphenylphosphine.

13. The improvement of claim 12 wherein said catalyst is a palladium bis(triphenylphosphine) dichloro complex.

14. The improvement of claim 13 wherein said catalyst is formed by adding to said IBPE separate amounts of palladium dichloride and triphenylphosphine.

15. In a method of preparing ibuprofen comprising carbonylating 1-(4′ isobutylphenyl)ethanol (IBPE) with carbon monoxide in an acidic aqueous medium and in the presence of (1) a catalyst consisting essentially of palladium-dihalide compound which is complexed with a phosphine ligand; (2) dissociated hydrogen ions; (3) dissociated halide ions; and (4) optionally a solvent, the improvement which comprises: recovering the palladium-phosphine catalyst complex by precipitating said complex from the organic phase formed from said carbonylation in which said organic phase contains the ibuprofen product.

16. The improvement of claim 15 wherein said phosphine ligand is a triorganophosphine.

17. The improvement of claim 16 wherein said ligand is triphenylphosphine.

18. The improvement of claim 17 wherein said catalyst is a palladium bis(triphenylphosphine) dichloro complex.

19. The improvement of claim 15 wherein the source of said hydrogen ions and halide ions is a hydrogen halide.

20. The improvement of claim 18 wherein said hydrogen halide is hydrogen chloride, hydrogen bromide, or hydrogen iodide.

21. In a method of preparing ibuprofen comprising carbonylating 1-(4′-isobutylphenyl)ethanol (IBPE) with carbon monoxide in an acidic aqueous medium and in the presence of (1) a catalyst consisting essentially of a palladium compound which is complexed with a phosphine ligand; (2) dissociated hydrogen ions; (3) dissociated halide ions; and (4) optionally a solvent, the improvement which comprises: recovering the palladium-phosphine catalyst complex by precipitating said complex from the organic phase formed from said carbonylation in which said organic phase contains the ibuprofen product, and recycling said recovered catalyst to said carbonylation.

22. The improvement of claim 21 wherein said phosphine ligand is a triorganophosphine.

23. The improvement of claim 21 wherein said ligand is triphenylphosphine.

24. The improvement of claim 21 wherein said catalyst is a palladium bis(triphenylphosphine) dichloro complex.

European Patent Office

# EUROPEAN SEARCH REPORT

Application Number

EP 89 30 3718

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| P,Y | EP-A-0 284 310 (HOECHST CELANESE) * Claims * | 1 | B 01 J 31/40<br>C 07 C 57/30<br>C 07 C 51/12 |
| Y | GB-A-2 085 747 (MITSUBISHI CHEMICALS) * Claim 1 * | 1 | |
| A | EP-A-0 073 341 (HOECHST) | | |
| A | EP-A-0 210 017 (HALCON) | | |
| A | US-A-4 605 541 (J. PUGACH) | | |

TECHNICAL FIELDS SEARCHED (Int. Cl.4)

B 01 J
C 07 C

The present search report has been drawn up for all claims

| Place of search | Date of completion of the search | Examiner |
|---|---|---|
| THE HAGUE | 26-06-1989 | THION M.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)